# EUROPEAN PATENT SPECIFICATION

(11) **EP 4 501 235 B1**
(45) Date of publication and mention of the grant of the patent: **06.05.2026**
(21) Application number: 23778418.6
(22) Date of filing: 31.03.2023
(51) Int. Cl.: A61B 5/293, A61B 5/291, A61N 1/05, A61B 5/369

(54) **FLEXIBLE NEURAL ELECTRODE COMPOSITE STRUCTURE AND MANUFACTURING METHOD THEREFOR, AND AUXILIARY IMPLANTATION ASSEMBLY**
FLEXIBLE VERBUNDSTRUKTUR MIT NEURALER ELEKTRODE SOWIE HERSTELLUNG DAFÜR SOWIE HILFSIMPLANTATIONSANORDNUNG
STRUCTURE COMPOSITE D'ÉLECTRODE NEURONALE FLEXIBLE ET SON PROCÉDÉ DE FABRICATION, ET ENSEMBLE D'IMPLANTATION AUXILIAIRE

(30) Priority: 02.04.2022 CN 202210351875
(43) Date of publication of application: 05.02.2025
(73) Proprietor: Beijing BCIFlex Medical Technology Co., Ltd., Beijing 100190 (CN)
(72) Inventor: FANG, Ying, Beijing 100190 (CN); TIAN, Huihui, Beijing 100190 (CN); FANG, Runjiu, Beijing 100190 (CN)
(74) Representative: Gulde & Partner
(86) International application number: PCT/CN2023/085296
(87) International publication number: WO 2023/186053

(56) References cited:
- CN-A- 101 884 530
- CN-A- 111 450 411
- CN-A- 111 990 995
- CN-A- 112 022 154
- CN-A- 114 259 234
- CN-A- 114 259 234
- CN-U- 209 252 829
- US-A1- 2020 261 025
- ZHENGTUO ZHAO ET AL: "Parallel, minimally-invasive implantation of ultra-flexible neural electrode arrays", JOURNAL OF NEURAL ENGINEERING, INSTITUTE OF PHYSICS PUBLISHING, BRISTOL, GB, vol. 16, no. 3, 30 April 2019 (2019-04-30), pages 35001, XP020335011, ISSN: 1741-2552, [retrieved on 20190430], DOI: 10.1088/1741-2552/AB05B6
- GUO ZHEJUN ET AL: "A Flexible and Stretchable Kirigami-Inspired Implantable Neural Probe with Floating Microsites for Electrophysiology Recordings", 2020 IEEE 33RD INTERNATIONAL CONFERENCE ON MICRO ELECTRO MECHANICAL SYSTEMS (MEMS), IEEE, 18 January 2020 (2020-01-18), pages 350 - 353, XP033753220, DOI: 10.1109/MEMS46641.2020.9056263

## Description

### CROSS-REFERENCE TO RELATED APPLICATION(S)

The present application is based on and claims the priority of China Patent Application No.202210351875.0 filed on April 2, 2022 and entitled "flexible neural electrode composite structure and manufacturing and implantation method therefor, and auxiliary implantation assembly".

### TECHNICAL FIELD

The present disclosure relates to a technical field of neuroscience, in particular to a flexible neural electrode composite structure, and a manufacturing method .

### BACKGROUND

In recent years, as an important tool for brain function analysis, brain disease treatment and brain-computer interface, implantable neural electrodes have been developed rapidly. However, since traditional implantable neural electrodes are rigid and are not matched with the mechanical properties of the brain tissue, there will be a relative movement between the neural electrode and the brain tissue under the influence of breathing and movement after the neural electrode is implanted in the brain, which will result in considerable damage to the brain tissue around the electrode and further cause inflammatory reactions. When a surface of the electrode is wrapped by immunoproliferative cells, the recorded signals of brain electrodes will be continuously weakened until failed. The paper "Parallel, minimally-invasive implantation of ultraflexible neural electrode arrays" provides a facile implantation method to apply ultraflexible neural probes in scalable neural recording. CN209252829U discloses a flexible implantable biosensor, an implantation needle for optoelectronic devices, and an implantation method thereof. CN114259234A relates to an auxiliary implantation device for high-throughput flexible electrodes and an assembly method thereof, wherein the auxiliary implantation device is used to assist in inserting the flexible electrodes into the brain of a living organism. The paper "A flexible and stretchable kirigami-inspired implantable neural probe with floating microsites for electrophysiology recordings" reports on a Nexible and stretchable neural probe based on polyimide (PI) which has excellent robustness and biocompatibility.

Compared with the rigid neural electrode, the flexible neural electrode has mechanical properties more matched with the nerve tissue, which can reduce immune damage to the brain tissue, thus improving the stability of detection of nerve signals and can be used for long-term recording of electroencephalogram (EEG) signals. However, how to realize high-throughput and large-scaled implantation of flexible neural electrodes in the brain (and other nerve tissues) is the problem to be solved urgently.

### SUMMARY

It is an object of the present disclosure to provide a flexible neural electrode composite structure, and a manufacturing method. The object is achieved by the features of the respective independent claims. Further embodiments are defined in the corresponding dependent claims. Even though the description refers to embodiments or to the invention, it is to be understood that the invention is defined by the claims and embodiments of the invention are those comprising at least all the features of one of the independent claims.

### BRIEF DESCRIPTION OF DRAWINGS

In order to explain the technical solution of the embodiments of the present disclosure more clearly, the accompanying drawings of the embodiments will be briefly introduced below. Obviously, the drawings in the following description only relate to some embodiments of the present disclosure, and are not intended to limit the present disclosure.
Fig. 1 is a schematic perspective view of a flexible neural electrode composite structure provided by an embodiment of the present disclosure.
Fig. 2 is a schematic cross-sectional view of the flexible neural electrode composite structure of Fig. 1 taken along dashed plane A.
Fig. 3 is a partially enlarged view of the flexible neural electrode composite structure in Fig. 2.
Fig. 4A is a partially three-dimensional structural diagram of region b of the flexible neural electrode composite structure of Fig. 1.
Fig. 4B is a top view of a flexible neural electrode array of Fig. 1.
Fig. 5 is a schematic structural diagram of an auxiliary implantation assembly provided by an embodiment of the present disclosure.
Fig. 6 is a schematic cross-sectional view of an auxiliary implantation assembly according to another embodiment of the present disclosure.
Fig. 7 is a schematic structural diagram of a flexible neural electrode composite structure provided by another embodiment of the present disclosure.
Fig. 8 is a flowchart of a manufacturing method of a flexible neural electrode composite structure provided by an embodiment of the present disclosure.
Fig. 9 is a schematic structural diagram of a flexible neural electrode formed in a manufacturing method of a flexible neural electrode composite structure provided by an embodiment of the present disclosure.
Fig. 10 is a schematic cross-sectional view taken along line AA of Fig. 9.
Figs. 11 to 13 are schematic cross-sectional views of a manufacturing method of an auxiliary implantation assembly provided by an embodiment of the present disclosure.
Fig. 14 is a schematic structural diagram of a composite structure assembly provided by the present disclosure.
Fig. 15 is an implantation method of flexible neural electrodes adopting the flexible neural electrode composite structure of Fig. 1, as provided by the present disclosure.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by one of ordinary skill in the art to which the present disclosure belongs. The terms "first," "second," etc., which are used in the present disclosure, are not intended to indicate any sequence, amount or importance, but distinguish various components. Also, the terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

In order to be combined with the nerve tissue closely and stably on a large spatial scale, it is necessary to prepare a flexible neural electrode array with high throughout and high coverage. However, the existing implantation process of flexible neural electrodes is restrained by lengthy implantation operation and limited electrode implantation area. For example, when the flexible neural electrodes in the array are implanted individually one by one, it is not only time-consuming and labor-intensive, but also liable to cause intraoperative and postoperative complications because of the long-time craniotomy during the operation.

Therefore, the embodiments of the present disclosure provide a flexible neural electrode composite structure, a manufacturing method and an implantation method thereof, a composite structure assembly and an implantation method thereof, and an auxiliary implantation assembly. By adopting the flexible neural electrode composite structure with auxiliary implantation assembly to realize high-throughput implantation, the implantation difficulty is reduced, and the operation time is shortened.

At least one embodiment of the present disclosure provides a flexible neural electrode composite structure, which includes: a plurality of flexible neural electrodes, wherein each flexible neural electrode includes an implant part and an auxiliary structure provided on the implant part; an auxiliary implantation assembly including a plurality of auxiliary implantation needles in one-to-one correspondence with the plurality of flexible neural electrodes, wherein each auxiliary implantation needle includes an auxiliary implantation end located close to one end of the corresponding flexible neural electrode, and the auxiliary implantation end is configured to be assembled with the auxiliary structure; and a fixture configured to fix the auxiliary implantation end and the auxiliary structure which have been assembled.

In the flexible neural electrode composite structure provided by the embodiment of the present disclosure, the auxiliary implantation end and the auxiliary structure are fixed by the fixture, so that the auxiliary implantation assembly and the plurality of flexible neural electrodes are fixed together. In this way, a plurality of flexible neural electrodes can be implanted into the target tissue at the same time during the implantation process of the flexible neural electrodes. Compared with the implantation method in which the electrodes are implanted individually one by one, on the one hand, it shortens the implantation time and reduces the implantation difficulty, thus realizing the implantation of a flexible neural electrode array in a high-throughput and high-coverage manner; on the other hand, before the flexible neural electrodes are implanted, the flexible neural electrodes and the auxiliary implantation assembly having been assembled are fixed or connected together by a fixture, so that the operation of on-site assembling the flexible neural electrodes with the auxiliary implantation assembly during implantation is omitted, the implantation efficiency is improved, and the operation time is shortened; in addition, because the flexible neural electrodes and the auxiliary implantation assembly have been assembled into an integrated structure, it is convenient for transportation and usage. In the embodiment of the present disclosure, "a plurality of " refers to two or more.

In the embodiment of the present disclosure, a plurality of flexible neural electrodes can be arranged in one or more rows, thereby forming a flexible neural electrode array. A plurality of auxiliary implantation needles can also be arranged in one or more rows, thereby forming an auxiliary implantation needle array. For example, the auxiliary implantation needle array is an optical fiber array, tungsten wire array, a platinum-iridium alloy wire array and a nickel-chromium alloy wire array obtained by three-dimensionally spatial arrangement; or a silicon needle array prepared by using a micro-electro-mechanical system (MEMS); or a comb-shaped array obtained by deep silicon etching; or a needle-like array obtained by MEMS processing.

The present disclosure will be explained by several specific embodiments. In order to keep the following description of the embodiments of the present disclosure clear and concise, detailed descriptions of known functions and known components may be omitted. When any component of an embodiment of the present disclosure appears in more than one drawing, the component may be represented by the same reference numeral in each drawing.

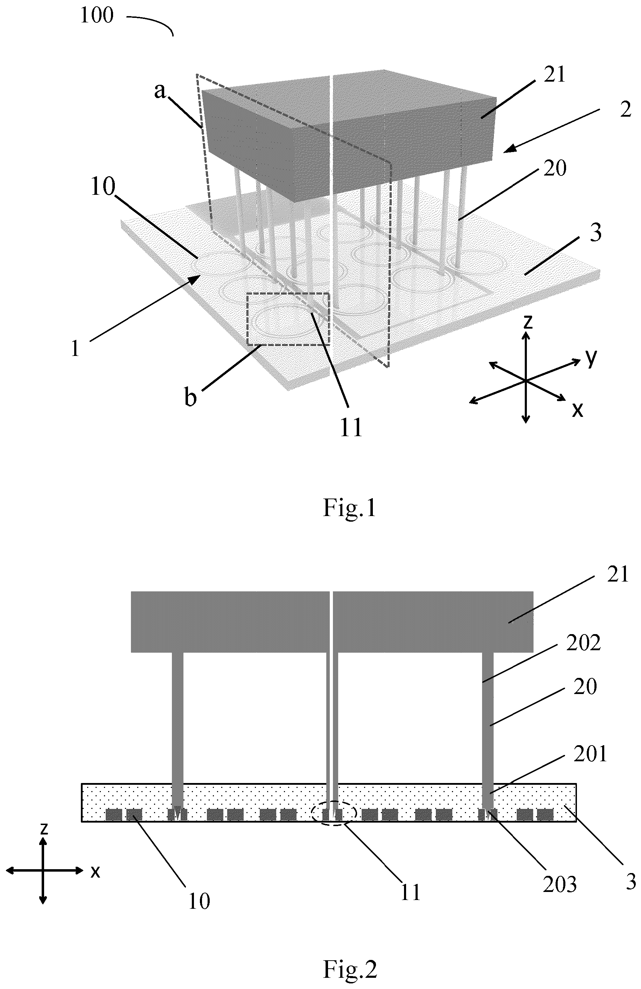

Fig. 1 is a schematic perspective view of a flexible neural electrode composite structure provided by an embodiment of the present disclosure.

As illustrated in Fig. 1, a flexible neural electrode composite structure 100 includes a plurality of flexible neural electrodes 1 (i.e., a flexible neural electrode array), an auxiliary implantation assembly 2 and a fixture 3.

For example, each flexible neural electrode 1 includes an implant part 10 and an auxiliary structure 11 provided on the implant part 10. The implant part 10 is configured to be implanted into a target tissue, such as brain tissue of a human or an animal, under the action of an external force.

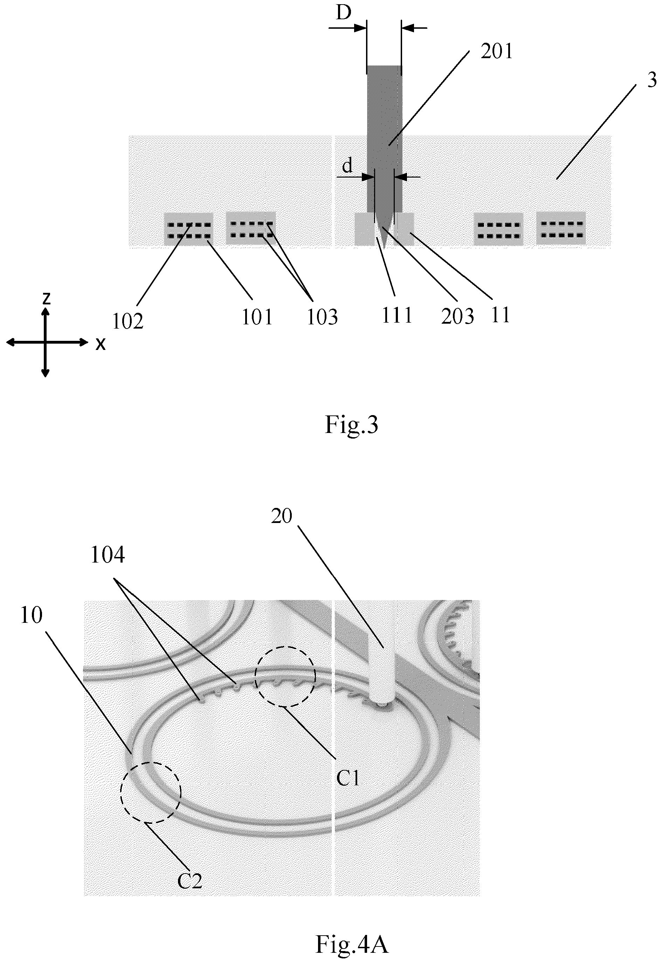

Fig. 2 is a schematic cross-sectional view of the flexible neural electrode composite structure of Fig. 1 taken along the dashed plane a. Fig. 3 is a partially enlarged view of the flexible neural electrode composite structure in Fig. 2.

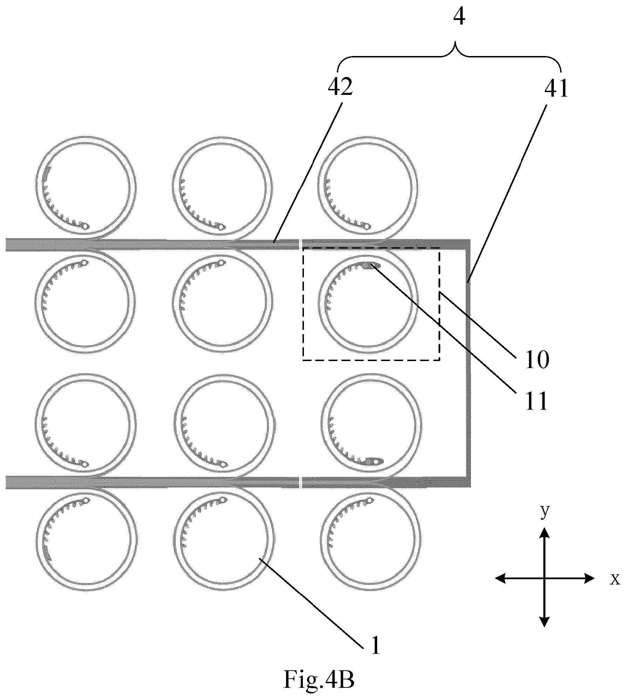

As illustrated in Fig. 3, the implant part 10 of the flexible neural electrode 1 includes, for example, a flexible insulating layer 101 and at least one conductive layer 102 buried in the flexible insulating layer 101. For example, the conductive layer 102 includes a plurality of conductive wires 103. The plurality of conductive wires 103 are insulated from each other by the flexible insulating layer 101. As illustrated in Fig. 4, the implant part 10 is provided with a plurality of electrode sites 104, which are connected with the plurality of conductive wires 103 in one-to-one correspondence for nerve recording or regulation. The more conductive wires 103, the more electrode sites 104 of the flexible neural electrode 1, and the wider the range of signal recording. In one example, the electrode sites 104 are formed by removing part of the flexible insulating layer 101 to expose the conductive wires 103.

Each implant part 10 in Figs. 3 and 4A only illustrates ten electrode sites 104 and ten conductive wires 103 connected thereto. It can be understood that in other embodiments of the present disclosure, the number of the electrode sites 104 and the number of the conductive wires 103 of each implant part 10 can be determined according to actual needs, which is not limited in the embodiment of the present disclosure.

In the embodiment of the present disclosure, the plurality of implant parts 10 of the plurality of flexible neural electrodes 1 can be arranged in various ways. Fig. 4B is a top view of a plurality of flexible neural electrodes of Fig. 1. As illustrated in Fig. 4B, the plurality of implant parts 10 are arranged in a 4x3 array. However, the embodiment of the present disclosure is not limited thereto, and the plurality of implant parts 10 can also be arranged in m rows and n columns, where m is greater than or equal to 1 and n is greater than or equal to 1.

In the flexible neural electrode array of Fig. 4B, the diameter of each spiral structure is about 1 mm. The width of each flexible neural electrode is about 15 µm, and each flexible neural electrode is distributed with 10 detection sites (electrode sites 104). The implant part is divided into five layers (insulating layer/conductive layer/insulating layer/conductive layer/insulating layer). The overall thickness of the flexible neural electrode is about 2.5 µm. The implant part 10 is located at a front end of each flexible neural electrode 1, and a rear end of each flexible neural electrode 1 is provided with a pad corresponding to each electrode site 104, and the pad is connected to an external circuit.

As illustrated in Figs. 2 and 3, the implant part 10 is provided with an auxiliary structure 11 configured to be connected with the auxiliary implantation assembly 2. For example, the auxiliary structure 11 and the auxiliary implantation assembly 2 are connected with each other by way of plugging-in.

As illustrated in Fig. 1, the auxiliary implantation assembly 2 includes, for example, a plurality of auxiliary implantation needles 20 in one-to-one correspondence with the plurality of flexible neural electrodes 1, and each auxiliary implantation needle 20 includes an auxiliary implantation end 201 and a fixing end 202, wherein the auxiliary implantation end 201 is located at one end of the auxiliary implantation needle 20 close to the flexible neural electrode 1, and the fixing end 202 is located at the other end of the auxiliary implantation needle 20 away from the flexible neural electrode 1. The auxiliary implantation end 201 is configured to be assembled with the auxiliary structure 11, so that the flexible neural electrode 1 and the auxiliary implantation assembly 2 can be assembled together.

In the embodiment of the present disclosure, the plurality of flexible neural electrodes 1 are connected with the auxiliary implantation assembly 2 by using the auxiliary implantation end 201 and the auxiliary structure 11 having been well assembled, which is beneficial to the batch implantation of a plurality of flexible neural electrodes 1 through the auxiliary implantation assembly 2, thereby realizing electrode implantation in a high-throughput and high-coverage way.

In the embodiment of the present disclosure, the extension direction of the plurality of auxiliary implantation needles 20 is not parallel to the extension direction of the plurality of implant parts 10 of the plurality of flexible neural electrodes 1. In other words, the extension direction of the plurality of auxiliary implantation needles 20 is spatially crossed with the extension direction of the plurality of implant parts 10 of the plurality of flexible neural electrodes 1, that is, they may or may not intersect with each other.

For example, as illustrated in Figs. 1 and 2, the plurality of auxiliary implantation needles 20 are parallel to each other and extend in the z direction. The implant part 10 of each flexible neural electrode 1 is, a stretchable spiral structure, and the plane where the spiral structure is located is the xy plane. The extension direction z of the auxiliary implantation needle 20 is not parallel to the xy plane where the spiral structure is located, for example, they are perpendicular to each other. In this way, when the flexible neural electrodes are implanted, by setting the extension direction of the plurality of auxiliary implantation needles 20 to be perpendicular to the extension direction of the plurality of implant parts 10 of the plurality of flexible neural electrodes 1, it is beneficial to controlling the implantation depth of the flexible neural electrodes, so that the flexible neural electrodes can reach the target tissue.

Figs. 1 and 2 only illustrate the case where the extension direction of the auxiliary implantation needle 20 is perpendicular to the extension direction of the implant part 10 of the flexible neural electrode. It can be understood that in other embodiments of the present disclosure, the extension direction of the auxiliary implantation needle 20 may have a certain inclination angle with respect to the xy plane where the spiral structure is located, for example, the inclination angle is greater than 0 degree and less than or equal to 90 degree, which is also beneficial to controlling the implantation depth of the flexible neural electrodes and is not limited in the embodiment of the present disclosure.

As illustrated in Fig. 1 and Fig. 4A, the implant part 10 of each flexible neural electrode 1 has a stretchable spiral structure, it is beneficial to unfolding the spiral structure by using the auxiliary implantation assembly 2 during the implantation of the flexible neural electrode 1, thus flexibly adjusting the implantation depth of the flexible neural electrode 1 and further reducing the implantation difficulty.

In the embodiment of the present disclosure, when the thickness of the spiral structure in the z direction is ignored, the spiral structure may be a two-dimensional planar structure as illustrated in Fig. 1, or a three-dimensional structure, that is, in a three-dimensional shape by spiralling along the z direction, which is not limited in the present disclosure. Further, when the spiral structure is a two-dimensional planar structure as illustrated in Fig. 1, the spiral structure has the shape of, for example, a circle, a triangle, a quadrilateral, a polygon, or a rounded triangle, a rounded quadrilateral, a rounded polygon, etc. Preferably, the spiral structure has the shape of a circle, which can be formed as a semicircle (i.e. 1/2 circle), a 2/3 circle or at least one circle, etc. Those skilled in the art can determine the number of turns of the spiral structure according to the actual needs of the implantation depth, which is not limited in the present disclosure. When the number of turns increases, the implantation depth or length can be increased.

In the embodiment of the present disclosure, the auxiliary structure 11 is located at the end of the spiral structure, that is, the terminal end of the implant part 10, which is beneficial to drawing or pulling the spiral structure to be unfolded more easily during the implantation of the flexible neural electrode. It can be understood that in other embodiments of the present disclosure, the auxiliary structure 11 can also be located at other positions at the end of the implant part 10 (for example, the C1 position between the two electrode sites 104 in Fig. 4A), or at a portion of the implant part 10 away from the terminal end (for example, the C2 position in Fig. 4A), which also enables the flexible neural electrode to be stretched or unfolded. Therefore, the position of the auxiliary structure 11 is not limited in the embodiment of the present disclosure.

Figs. 1 and 2 only illustrate that the implant part 10 is a stretchable spiral structure, and it can be understood that the implant part 10 can also have other shapes in the present disclosure, such as a linear structure or a wavy structure. The implant part 10 may be one or more of a spiral structure, a wavy structure and a linear structure.

In the embodiment of the present disclosure, the auxiliary implantation end 201 and the auxiliary structure 11 are detachably assembled together, for example, by way of plugging-in, which is convenient for assembly and disassembly.

For example, as illustrated in Fig. 2, the auxiliary implantation end 201 and the auxiliary structure 11 are assembled together by way of plugging-in. The auxiliary structure 11 is, for example, a through hole 111 located on the implant part 10. To facilitate inserting into the through hole 111, the auxiliary implantation end 201 includes a tip 203, and the diameter of the tip 203 is smaller than or equal to that of the auxiliary implantation needle 20.

For example, the tip 203 has a cross section that gradually decreases towards the auxiliary structure 11, for example, cone shape as illustrated in the figure, which may be a circular cone or a pyramid. In this way, when assembling the auxiliary implantation end 201 with the auxiliary structure 11, the tip 203 can serve for guiding, which is beneficial to quickly inserting the auxiliary structure 11 for assembling. The tip 203 may also be flat-headed, and the shape of the tip 203 is not specifically limited in the embodiment of the present disclosure.

Fig. 4A only illustrates the case where the auxiliary structure 11 is a through hole. In some other embodiments of the present disclosure, the auxiliary structure 11 can also be a groove or a protrusion, and any structure that can be plugged-in with the auxiliary implantation end 201 is included in the embodiment of the present disclosure.

In the embodiment of the present disclosure, the auxiliary implantation end 201 is partially or completely inserted into the auxiliary structure 11. For example, when the auxiliary implantation end 201 is completely inserted into the auxiliary structure 11, the firmness after assembling can be improved; when the auxiliary implantation end 201 is partially inserted into the auxiliary structure 11, the separation of the flexible neural electrode 1 from the auxiliary implantation assembly 2 after the flexible neural electrode 1 has been implanted can be facilitated. As illustrated in Fig. 3, the auxiliary implantation end 201 is partially inserted into the through hole 111, which means that the tip 203 is inserted into the through hole 111.

In the embodiment of the present disclosure, the shape of the cross-section of each auxiliary implantation needle 20 includes one of a triangle, a rectangle, a circle, an ellipse and a regular polygon. The shape of the cross-section of the auxiliary implantation needle 20 refers to the shape of the cross-section in the xy plane of the auxiliary implantation needle 20 in Fig. 1. When the shape of the cross-section is a circle or an ellipse, it can reduce the damage to brain tissue and hence is preferred.

In the embodiment of the present disclosure, the material of each auxiliary implantation needle 20 includes one or more of metal, alloy and nonmetal. The metal includes, for example, tungsten. The nonmetal includes silicon or silicon dioxide, for example. The alloy includes, for example, platinum-iridium alloy or nickel-chromium alloy, or the like.

For example, the auxiliary implantation needle 20 is, for example, a rigid micro-wire, which is obtained by processing an optical fiber or a tungsten wire with good collimation; or a silicon-based needle-like array obtained by deep silicon etching through micro-electro-mechanical system (MEMS) technology; or a SU-8 needle-like array structure with high aspect ratio which is obtained by MEMS technology. Further, when optical fiber or tungsten wire is used, not only the strength is higher, but also the collimation is better.

In the embodiment of the present disclosure, when the shape of the cross-section of the auxiliary implantation needle 20 is a circle, the diameter (the diameter D illustrated in Fig. 3) of the auxiliary implantation needle 20 is 5 µm to 200 µm, for example, it may be 5 µm, 10 µm, 50 µm, 100 µm, 150 µm, and 200 µm. If the diameter D is larger, the damage to brain tissue is greater. In one example, the diameter D is 50 µm to 150 µm, for example, it may be 50 µm, 75 µm, 100 µm, 125 µm and 150 µm; further, it is, for example, 75 µm to 100 µm. The auxiliary implantation needle 20 is formed by an etching process, for example.

In the embodiment of the present disclosure, the maximum diameter d of the tip of the auxiliary implantation needle 20 is 1 µm to 100 µm, for example, it may be 1 µm, 10 µm, 50 µm and 100 µm. In one example, the maximum diameter d is 10 µm to 100 µm, e.g., 10 µm, 20 µm, 50 µm and 100 µm; further, it is, for example, 20 µm to 50 µm.

In the embodiment of the present disclosure, it's preferred for the difference between the diameter of the tip 203 of the auxiliary implantation needle 20 and diameter of the auxiliary implantation needle 201 to be greater, for example, the difference between the diameters of them is 20 µm to 50 µm.

In the embodiment of the present disclosure, the diameter of the through hole 111 is 1 µm to 100 µm, for example, it may be 1 µm, 10 µm, 50 ttm and 100 µm. In one example, the diameter of the through hole 111 is 5 µm to 100 µm, e.g., 5 µm, 10 µm, 20 µm, 50 µm and 100 µm; further, it is, for example, 20 µm to 50 µm. In the embodiment of the present disclosure, in order to ensure the fitting between the tip 203 and the through hole 111, the diameter of the through hole 111 is greater than or equal to the diameter d of the tip, and is less than or equal to the diameter D of the auxiliary implantation needle.

As illustrated in Figs. 1 to 3, the fixture 3 is configured to fix the auxiliary implantation end 201 and the auxiliary structure 11 which have been assembled. For example, at least a portion of the fixture 3 is located between the auxiliary implantation end 201 and the auxiliary structure 11 to maintain the relative position between the auxiliary implantation end 201 and the auxiliary structure 11, which can ensure that the flexible neural electrode 1 and the auxiliary implantation assembly 3 will not move relative to each other during transportation. Moreover, before the implantation of the flexible neural electrode, the flexible neural electrode 1 and the auxiliary implantation assembly 3 having been assembled are fixed or connected together by using the fixture 3, so that the operation of on-site assembling the flexible neural electrode 1 with the auxiliary implantation assembly 3 during implantation is omitted, the implantation efficiency is improved, and the operation time is shortened; in addition, because the flexible neural electrodes 1 and the auxiliary implantation assembly 3 are assembled into an integrated structure, it is convenient for transportation and usage.

In the embodiment of the present disclosure, the fixture 3 is configured such that its physical state changes with the change of external conditions. For example, at least one physical parameter, such as volume, for characterizing the physical state is changed with the change of external conditions. In one example, the fixture 3 can be changed from a solid state to a liquid state by changing the illumination or the ambient temperature, which results in the change of volume. In another example, when a specific liquid is dropped onto the fixture 3, although the fixture 3 remains solid, its volume is changed from small to large (that is, the swelling phenomenon occurs). When the physical state of the fixture 3 is changed, the auxiliary implantation assembly 2 and the flexible neural electrode 1 are no longer bound together by the fixture 3 but are separable, which is convenient for the subsequent operation of implanting the flexible neural electrode.

In the embodiment of the present disclosure, the fixture 3 includes one or more of a photo-meltable material, a thermal-meltable material, a liquid-swellable material and a liquid-dissolvable material.

For example, the photo-meltable material includes positive photosensitive resin, such as diazonaphthaquinone-based photosensitive resin, the working principle of which is that the exposed part of the photosensitive film is decomposed and denitrified upon being exposed to light, and generates acid with water through rearrangement reaction of molecules.

For example, the thermal-meltable material includes thermal-meltable polymer, such as one or more of polyethylene glycol (PEG) and polylactic acid-glycolic acid copolymer-polyethylene glycol (PLGA-PEG).

For example, the liquid-swellable material includes water-swellable polymer, such as one or more of poly (ethylene glycol) alginate diacrylate (PEGDA) and polyacrylamide-alginate (PAAm).

For example, the liquid-dissolvable material includes water-dissolvable polymer, such as one or more of polyvinyl alcohol (PVA), silk protein, polyethylene glycol (PEG), polylactic acid-glycolic acid copolymer-polyethylene glycol (PLGA-PEG) and gelatin; the liquid includes one or more of ultrapure water, physiological saline and phosphate buffer solution (PBS). When PVA solution is selected, the concentration of PVA solution is 2%~10%, such as 2%, 5%, 10%, etc., for example 5%.

As illustrated in Figs. 1 to 3, the fixture 3 takes the form of a film, for example, a plurality of auxiliary implantation ends 201 and a plurality of auxiliary structures 11 are fixed together by the film. In this way, the process of forming the fixture 3 can be simplified, that is, a plurality of auxiliary implantation ends 201 and a plurality of auxiliary structures 11 may be fixed together by one-step film forming process. For example, at least a plurality of joints between a plurality of auxiliary implantation ends 201 and a plurality of auxiliary structures 11 can be fixed together through the film. This can reduce the usage amount of the fixing material and reduce the influence on the target tissue due to larger usage amount. For example, the thickness of the film is greater than the width of the joint in the z direction, for example, the range of the thickness is 30 µm to 50 µm.

As illustrated in Figs. 1 and 2, the auxiliary implantation assembly 2 further includes an auxiliary fixing member 21, which is provided at one side of the plurality of auxiliary implantation needles 20 away from the plurality of flexible neural electrodes 1 and is configured to fix the plurality of auxiliary implantation needles 20. By providing the auxiliary fixing member 21, the plurality of flexible neural electrodes 1 may be connected to the same object, so that a surgeon can move or implant the plurality of flexible neural electrodes 1, as a whole, by grasping the auxiliary fixing member during the implantation.

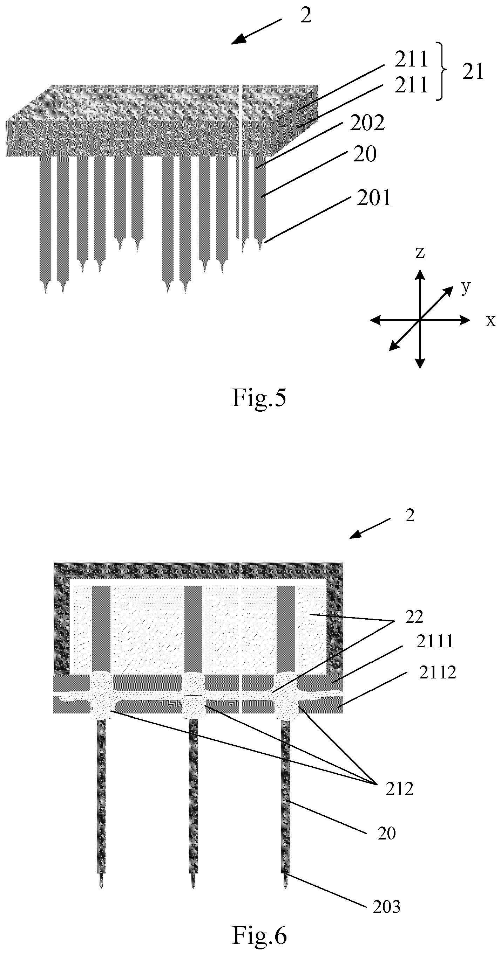

Fig. 5 is a schematic structural diagram of an auxiliary implantation assembly provided by an embodiment of the present disclosure.

As illustrated in Fig. 5, for example, the auxiliary fixing member 21 includes two auxiliary fixing plates 211, the plane where the auxiliary fixing plates 211 are located is a xy plane, and the extension direction of the plurality of auxiliary implantation needles 20 is a z direction, and thus the xy plane where the auxiliary fixing plates 211 are located is perpendicular to the extension direction z of the plurality of auxiliary implantation needles 20. By setting the plane where the auxiliary fixing plates 211 are located and the extension direction of the plurality of auxiliary implantation needles 20 to be perpendicular to each other, it is beneficial to controlling the implantation depth of the flexible neural electrode conveniently in the process of implanting the flexible neural electrode.

Fig. 5 only illustrates the case in which the plane where the auxiliary fixing plates 211 are located is perpendicular to the extension direction of the plurality of auxiliary implantation needles 20. It would be understood that in some other embodiments of the present disclosure, the extension direction of the plurality of auxiliary implantation needles 20 may not be parallel to the xy plane where the auxiliary fixing plates 211 are located. For example, the extension direction of the plurality of auxiliary implantation needles 20 has a certain inclination angle relative to the xy plane, and the inclination angle is, for example, more than 0 degree and less than or equal to 90 degree, which is also beneficial to controlling the implantation depth of the flexible neural electrode, and the embodiment of the present disclosure is not limited thereto.

For example, the auxiliary fixing plate 211 is detachably or fixedly connected with a plurality of auxiliary implantation needles 20. When a detachable connection is adopted, the number and positions of the auxiliary implantation needles 20 fixed to the auxiliary fixing plate 211 can be selected according to actual needs. When a fixed connection is adopted, the stability of the whole flexible neural electrode composite structure can be improved.

Fig. 6 is a schematic cross-sectional view of an auxiliary implantation assembly according to another embodiment of the present disclosure.

As illustrated in Fig. 6, the auxiliary fixing plate 211 is provided with openings 212, and the plurality of auxiliary implantation needles 20 each include a fixing end 202 away from the auxiliary implantation end 201 along the extension direction of the auxiliary implantation needle, and the fixing end 202 is configured to pass through the openings 212 to be connected with the auxiliary fixing plate 211. By providing the openings 212 in the auxiliary fixing plate 211, it is beneficial to realizing the detachable connection between the auxiliary implantation needles 20 and the auxiliary fixing plate 211.

As illustrated in Fig. 6, the auxiliary implantation assembly 2 further includes an adhesive 22, and at least part of the adhesive 22 is located between the auxiliary fixing plate 211 and the plurality of auxiliary implantation needles 20 so that the auxiliary fixing plate 211 is bonded with the plurality of auxiliary implantation needles 20. By providing the adhesive 22, the firmness between the auxiliary fixing plate 211 and the plurality of auxiliary implantation needles 20 can be improved, and the auxiliary implantation needles 20 can be prevented from falling off or deviating during the movement of the flexible neural electrode composite structure which may affect the implantation effect.

Fig. 6 illustrates the case where two auxiliary fixing plates 211 are provided, but it can be understood that the number of the auxiliary fixing plates 211 may also be one or more than two, which is not limited in the embodiment of the present disclosure. In this embodiment, the two auxiliary fixing plates 211 are provided with a gap therebetween, which can further ensure the collimation of the auxiliary implantation needles, and further improve the firmness between the auxiliary fixing plates 211 and the plurality of auxiliary implantation needles 20.

In the embodiment of the present disclosure, a plurality of auxiliary implantation needles 20 can be arranged in various ways. For example, in Fig. 5, the plurality of auxiliary implantation needles 20 are arranged in an array. However, in some other embodiments, the arrangement of the plurality of auxiliary implantation needles 20 may be determined depending on the arrangement of the plurality of flexible neural electrodes. For example, when the plurality of flexible neural electrode arrays are arranged in a circle, a square or a polygon, the auxiliary implantation needle array can also be presented as a circle, a square or a polygon so that the auxiliary implantation needles can be in one-to-one correspondence with the plurality of flexible neural electrodes 1.

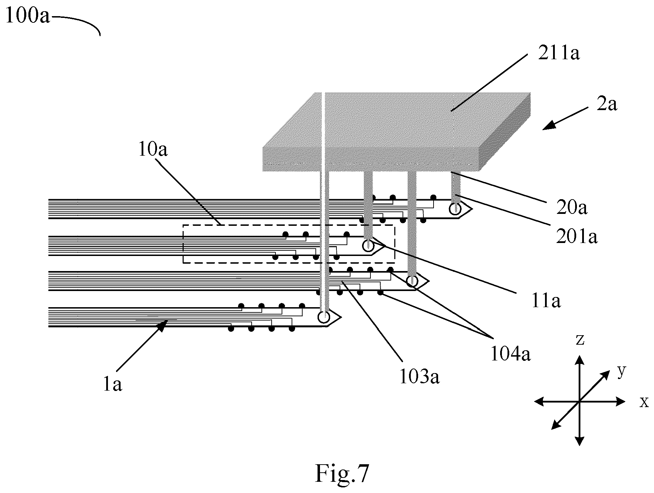

Fig. 7 is a schematic structural diagram of a flexible neural electrode composite structure provided by another embodiment of the present disclosure. As illustrated in Fig. 7, the flexible neural electrode composite structure 100a includes four flexible neural electrodes 1a, an auxiliary implantation assembly 2a and a fixture (not illustrated). The four flexible neural electrodes 1a are arranged in a 2x2 array.

For example, each flexible neural electrode 1a includes an implant part 10a and an auxiliary structure 11a provided on the implant part 10a. The implant part 10a is configured to be implanted into a target tissue under the action of an external force. For the specific structure of the implant part 10a of the flexible neural electrode 1a, reference can be made to the description of the previous embodiment, which will not be repeated here.

As illustrated in Fig. 7, the implant part 10a is provided with eight electrode sites 104a, and the eight electrode sites 104a are connected with eight conductive wires 103a in one-to-one correspondence for nerve recording or regulation. Compared with the electrode sites 104 located at one side of the implant part 10 in Fig. 4A, the electrode sites 104a in Fig. 7 are distributed at both sides of the implant part 10a, so that the number of electrode sites can be increased in a unit area, and more signals can be recorded or regulated.

As illustrated in Fig. 7, each implant part 10a has a linear structure, the extension direction of a plurality of auxiliary implantation needles 20 is the z direction, and the extension direction of a plurality of linear structures is the x direction, and thus the extension direction of the plurality of auxiliary implantation needles 20a is perpendicular to the extension direction of the plurality of linear structures. In this way, when the flexible neural electrode is implanted, by setting the extension direction of the plurality of auxiliary implantation needles 20a to be perpendicular to the extension direction of the plurality of implant parts 10a of the plurality of flexible neural electrodes 1a, it is beneficial to controlling the implantation depth of the flexible neural electrode, so that the flexible neural electrode can reach the target tissue.

Fig. 7 only illustrates the case where the extension direction of the auxiliary implantation needle 20a is perpendicular to the extension direction of the implant part 10a of the flexible neural electrode 1a, but it can be understood that in some other embodiments of the present disclosure, the extension direction of the auxiliary implantation needle 20a may have a certain inclination angle with respect to the extension direction of the linear structure, for example, the inclination angle is greater than 0 degree and less than or equal to 90 degree, which is also beneficial to controlling the implantation depth of the flexible neural electrode and is not limited in the embodiment of the present disclosure.

In the present disclosure, a plurality of flexible neural electrodes 1 may include a plurality of implant parts 10; some of these implant parts are linear structures as illustrated in Fig. 7, and some others of these implant parts are stretchable spiral structures as illustrated in Fig. 1. In this way, different requirements for implanted electrodes can be satisfied during the surgery.

In the present disclosure, the shape of the implant part can be varied, only the linear structure and the spiral structure are illustrated above, and other structures such as spring structure and net structure can also be included. In addition, the shape of the spiral structure is not limited to the circle illustrated above, but also may be a triangle, a quadrangle, a polygon or a rounded triangle, a rounded quadrangle, a rounded polygon, and the like.

Returning to Fig. 4B, the flexible neural electrode composite structure 100 may further include a support assembly 4. The support assembly 4 is connected with a plurality of implant parts of a plurality of flexible neural electrodes 1 and serves for supporting, thereby facilitating overall transfer of the plurality of flexible neural electrodes 1. The support assembly 4 includes a first support member 41 and a second support member 42 connected to the first support member 41, wherein the first support member 41 includes the flexible insulating layer 101 and the conductive layer 102 of the flexible neural electrode 1. The second support member 42 only includes the flexible insulating layer 101, and serves for insulating.

At least one embodiment of the present disclosure also provides a manufacturing method of the flexible neural electrode composite structure.

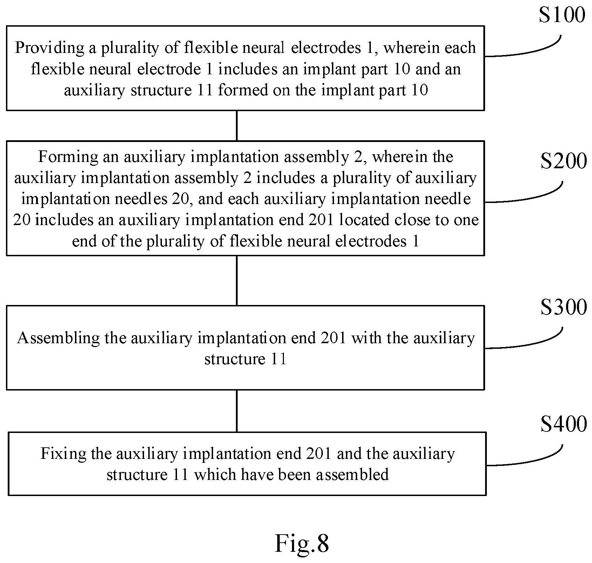

Fig. 8 is a flowchart of the manufacturing method of the flexible neural electrode composite structure provided by the embodiment of the present disclosure. As illustrated in Fig. 8, the manufacturing method of the flexible neural electrode composite structure 100 of Fig. 1 provided by the embodiment of the present disclosure includes the following steps:
S100: providing a plurality of flexible neural electrodes 1, wherein each flexible neural electrode 1 includes an implant part 10 and an auxiliary structure 11 formed on the implant part 10;
S200: forming an auxiliary implantation assembly 2, wherein the auxiliary implantation assembly 2 includes a plurality of auxiliary implantation needles 20, and each auxiliary implantation needle 20 includes an auxiliary implantation end 201 located close to one end of each of the plurality of flexible neural electrodes 1;
S300: assembling the auxiliary implantation end 201 with the auxiliary structure 11; and
S400: fixing the auxiliary implantation end 201 and the auxiliary structure 11 which have been assembled.

In the manufacturing method of the flexible neural electrode composite structure provided by the embodiment of the present disclosure, by fixing the auxiliary implantation assembly and a plurality of flexible neural electrodes together, the plurality of flexible neural electrodes can be implanted into the target tissue at the same time during the implantation process of the flexible neural electrodes. Compared with the implantation method in which the electrodes are implanted individually one by one, on the one hand, it shortens the implantation time and reduces the implantation difficulty, thus realizing the implantation of a flexible neural electrode array in a high-throughput and high-coverage manner; on the other hand, before the flexible neural electrodes are implanted, the flexible neural electrodes and the auxiliary implantation assembly having been assembled are fixed or connected together by a fixture, so that the operation of on-site assembling the flexible neural electrodes with the auxiliary implantation assembly during implantation is omitted, the implantation efficiency is improved, and the operation time is shortened; in addition, because the flexible neural electrodes and the auxiliary implantation assembly have been assembled into an integrated structure, it is convenient for transportation and usage.

For example, in step S100, there are various methods for forming the flexible neural electrode 1, such as photolithography, micro-electro-mechanical system (MEMS) and the like.

In one example, the preparation method of a plurality of flexible neural electrodes includes the following steps:
S101: cleaning and drying a base substrate.

For example, the base substrate is a silicon wafer, which is cleaned by ultrasonic wave and blow-dried by nitrogen gas, and then is cleaned by plasma.

S102: forming a plurality of grooves on the base substrate, wherein the plurality of grooves correspond to a plurality of auxiliary structures 11 on a plurality of implant parts 10 of a plurality of flexible neural electrodes 1.

For example, the auxiliary structure 11 may be a through hole, a groove or a protrusion. By providing the plurality of grooves, it is convenient to provide an accommodation space for the auxiliary implantation end 201 passing through a through hole when the auxiliary implantation needle 20 is inserted into the through hole, facilitating the transfer of electrodes.

S103: forming a sacrificial layer on the base substrate formed with the plurality of grooves.

When assembling the flexible neural electrode array with the auxiliary implantation assembly, the sacrificial layer is formed on the base substrate, which is beneficial to releasing the flexible neural electrode array formed on the base substrate. For example, one or more of polymethyl methacrylate (PMMA), aluminum (Al), nickel (Ni) and the like may be adopted for the sacrificial layer. In this embodiment, PMMA is adopted for the sacrificial layer, and the flexible neural electrode array is formed on the sacrificial layer. When the flexible neural electrode array is partially immersed in a solution such as acetone, the solution submerges the PMMA sacrificial layer, and the flexible neural electrode array can be released from the base substrate after the PMMA sacrificial layer is completely dissolved.

S104: forming a flexible neural electrode array on the base substrate formed with the sacrificial layer.

For example, the flexible neural electrode array includes a plurality of flexible neural electrodes 1. The flexible neural electrode 1 includes a flexible insulating layer 101 and a conductive layer 102, wherein the conductive layer 102 includes a plurality of conductive wires 103; the flexible neural electrode 1 further includes an implant part 1 and an auxiliary structure 11 located on the implant part 1. An electrode site 104 is formed by removing part of the flexible insulating layer 101 to expose part of the conductive wire 103. For example, the conductive layer 102 may be formed by a photolithography process.

In the embodiment of the present disclosure, the photolithography process includes, but is not limited to, coating a photoresist, exposing with a mask, developing, etching, lifting off the remaining photoresist, and the like. It can be understood that the conductive layer 102 may be a single-layer structure or a multi-layer structure, and each conductive layer 102 may be provided with a plurality of conductive wires 103, and adjacent conductive layers 102 are insulated from each other.

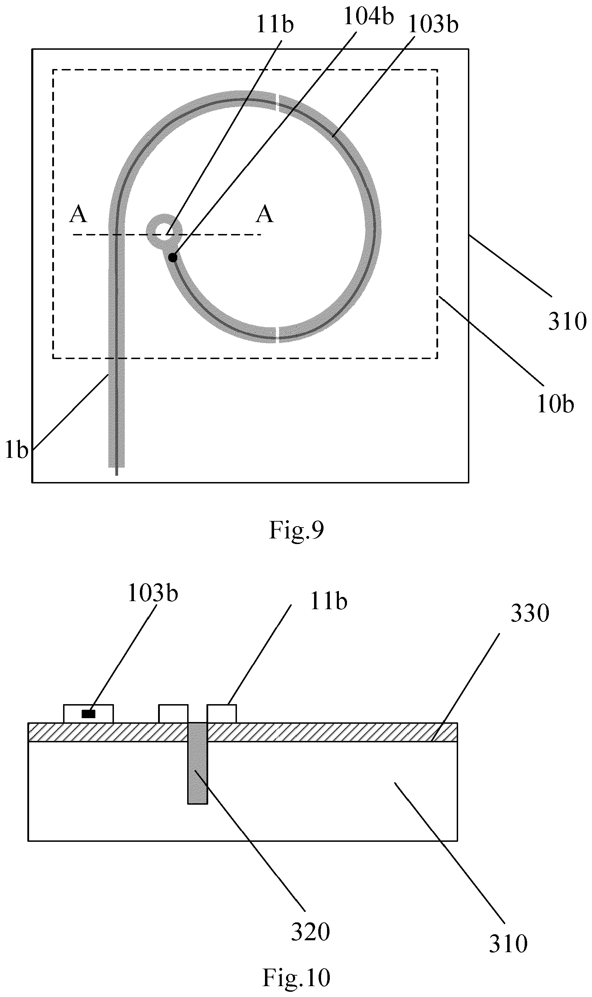

Fig. 9 is a schematic structural diagram of a flexible neural electrode formed in the manufacturing method of the flexible neural electrode composite structure provided by the embodiment of the present disclosure. Fig. 10 is a schematic cross-sectional view taken along line AA of Fig. 9.

As illustrated in Figs. 9 and 10, a circular groove 320 with a diameter of 60 µm and a depth of 100 µm is formed in a silicon wafer 310 to accommodate an auxiliary implantation needle passing through an auxiliary structure 11b. A PMMA sacrificial layer 330 is formed on the silicon wafer 310 where the circular groove 320 is formed. A flexible neural electrode 1b is formed on the PMMA sacrificial layer 330. The flexible neural electrode 1b includes an implant part 10b having a spiral structure. The implant part 10b includes an auxiliary structure 11b (a through hole 111b as illustrated in the figure) and an electrode site 104a. The flexible neural electrode 1b further includes a conductive wire 103b, which is connected to the electrode site 104a.

In the embodiment of the present disclosure, the diameter of the circular groove may be 5 µm to 100 µm, such as 5 µm, 10 µm, 20 µm, 25 µm, 30 µm, 40 µm, 50 µm, 100 µm, etc., for example, 40 µm to 60 µm; the depth of the circular groove may be 1 µm to 200µm, such as 0 µm, 5 µm, 10 µm, 15 µm, 25 µm, 30 µm, 40 µm, 50 µm, 100 µm, 150 µm, 200 µm, etc., for example, 100 µm to150 µm. The thickness of the sacrificial layer is 0 µm to 20 µm, such as 1 µm, 2 µm, 5 µm, 10 µm, 20 µm, etc., for example, 1 µm.

For example, step S200 may further include:
S201: forming an auxiliary fixing member 21;
S202: forming a plurality of auxiliary implantation needles 20;
S203: connecting the auxiliary fixing member 21 and the plurality of auxiliary implantation needles 20 together.

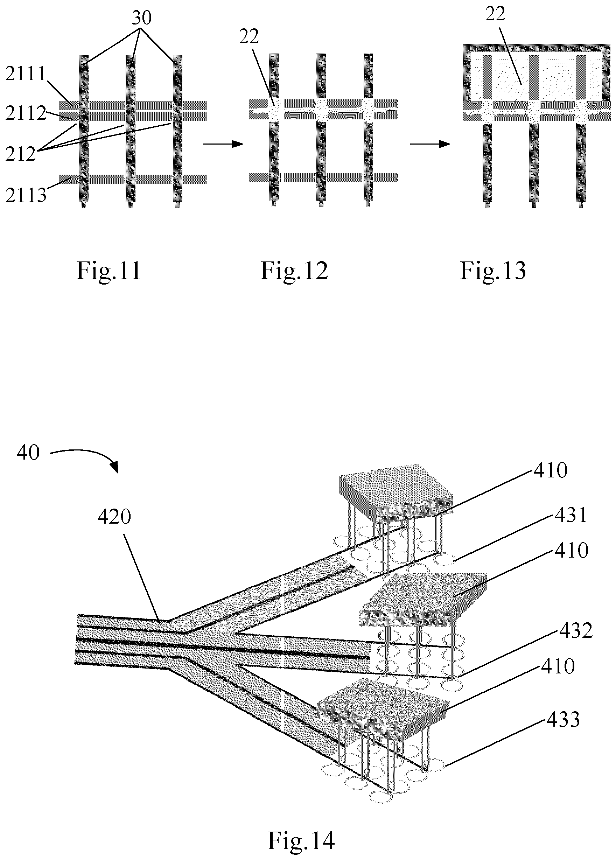

Figs. 11 to 13 are schematic cross-sectional views of a manufacturing method of the auxiliary implantation assembly provided by the embodiment of the present disclosure. For example, Figs. 11 to 13 illustrate a manufacturing method of the auxiliary implantation assembly of Fig. 6, including the following steps:
(1) A plurality of commercially available optical fibers 30 with a diameter of 250 µm are selected, and each optical fiber 30 consists of a high-purity silica (SiO2) core with a diameter of 125 µm and an acrylate coating layer with an outer diameter of 250 µm. The optical fiber 30 is cut into optical fiber segments with a length of about 1 cm by using an optical fiber cutter, and a portion of the acrylate coating layer with a thickness of about 50 µm at the tip of the optical fiber is stripped off by using a wire stripper.
(2) As illustrated in Fig. 11, three silicon wafers 2111-2113 are selected as auxiliary fixing plates, and a plurality of openings 212 are formed on each silicon wafer. For example, the silicon wafer 2111 is etched by laser to obtain the plurality of openings 212 arranged in a 3×4 array. The 3×4 array of openings has an arrangement as same as that of 3×4 array of through holes 111 in the flexible neural electrode array. The diameter of the opening 212 is, for example, 260 µm. Then, the plurality of optical fibers 30 are inserted into the corresponding twelve openings 212 of the silicon wafers 2111 to 2113 for fixing and collimation. The silicon wafers 2111 and 2112 serve to collimate the plurality of optical fibers 30, and the silicon wafer 2113 is used to fix the positions of the optical fibers 30.
(3) As illustrated in Fig. 12, an adhesive 22, such as epoxy glue, is added between two adjacent silicon wafers 2111 and 2112, and between the optical fiber 30 and each silicon wafer 2111 and 2112 for fixation. As illustrated in Fig. 13, the top of the silicon wafer 2111 is fixed in a 3D printed carrier with epoxy glue.
(4) As illustrated in Fig. 13, the tip of the optical fiber 30 is etched with buffered oxide etch (BOE) solution. The etching process is provided as follows: firstly, a core having a diameter of 125 µm at the tip of the optical fiber 30 is etched to a diameter of about 60 µm; then, the optical fiber 30 is taken out of the etch solution, washed with deionized water for three times, and immerged in anhydrous ethanol at 50°C to remove the acrylate coating layer on the surface of the optical fiber 30; finally, the optical fiber 30 from which the acrylate coating layer has been removed is etched with the BOE solution to obtain an optical fiber with a tip diameter of about 20 µm, and the auxiliary implantation assembly illustrated in Fig. 6 is obtained ultimately.

In the above-described manufacturing method, instead of the optical fiber 30, a tungsten wire may also be used as the auxiliary implantation needle. In this case, the above step 4) can be replaced by the following step 4'):

The front end with a length of about 6 mm of the tungsten wire is immersed in 2 mol/L sodium hydroxide (NaOH) solution, and an electrochemical etching process is carried out under a constant potential mode of Chenhua Electrochemical Workstation (CHI660) with an overpotential of 5.0 V to etch the tungsten wire until it has a diameter of about 100 µm; then the tip of the tungsten wire is lifted, and only a portion with a length of about 2 mm of the tip of the tungsten wire is immersed in the NaOH solution for further etching until the meniscus is broken, so as to obtain tungsten wire with a flat tip; finally, the auxiliary implantation assembly illustrated in Fig. 6 is also obtained.

For example, before step S300, the manufacturing method further includes:
S500: aligning the auxiliary implantation end 201 with the auxiliary structure 11.

For example, at least one auxiliary implantation end 201 among a plurality of auxiliary implantation ends 201 is aligned with at least one auxiliary structure 11 among a plurality of auxiliary structures 11, which is beneficial to assembling the auxiliary implantation end 201 with the auxiliary structure 11 more quickly.

For example, the auxiliary implantation assembly 2 is pre-fixed by using a stereo locator, and the array of flexible neural electrodes 1 is placed on a rotary stage and adjusted to be aligned with the auxiliary implantation assembly 2. The rotary stage is, for example, a multi-axis precision air-floated rotary stage that can rotate at multiple angles.

For example, the step S400 may further include: forming a fixture 3 at least at the joint between the auxiliary implantation end 201 and the auxiliary structure 11, as illustrated in Figs. 2 and 3. For example, the fixture 3 is at least filled between the tip 203 of the auxiliary implantation end 201 and the through hole 111, so as to maintain the relative position between the tip 203 and the through hole 111 unchanged. Further, for example, the fixture 3 is formed as a film that connects the plurality of auxiliary implantation ends 201 and the plurality of auxiliary structures 11 together. The specific material of the fixture 3 can refer to the description of the previous embodiments, and will not be described here.

In one example, after the auxiliary implantation assembly and the flexible neural electrode array are formed, the following steps are performed:
a) Placing a silicon wafer 310 formed with circular grooves 320, a PMMA sacrificial layer 330 and a plurality of flexible neural electrodes 1b (hereinafter referred to as flexible neural electrode array) on a multi-axis precision air-floated rotary stage;
b) Fixing the top of the auxiliary implantation assembly 2 prepared in Fig. 6 on a three-dimensionally movable stereo locator;
c) Adjusting the stereo locator to descend the auxiliary implantation assembly 2 to gradually approach the surface of the flexible neural electrode array, and adjusting the X, Y and Z axes of the air-floated rotary stage to align the tips of the plurality of optical fibers 30 of the auxiliary implantation assembly 2 with the plurality of through holes 111b in the flexible neural electrode array;
d) Continuing to descend the auxiliary implantation assembly 2 until the tips of the plurality of optical fibers 30 are inserted into the plurality of circular grooves 320 of the silicon wafer 310;
d) Dropping 5% PVA water solution on the tips of the optical fibers 30 and the surface of the flexible neural electrode array so that the flexible neural electrode array is fixed with the auxiliary implantation assembly 2 through the PVA after the water in the solution evaporates; and
f) Partially immerging the flexible neural electrode array in acetone for release, wherein the amount of the acetone is sufficient to submerge the PMMA sacrificial layer, and the flexible neural electrode array is released after the PMMA sacrificial layer is completely dissolved, thereby obtaining the flexible neural electrode composite structure.

At least one embodiment of the present disclosure also provides a composite structure assembly including a plurality of flexible neural electrode composite structures described in any of the previous embodiments.

Fig. 14 is a schematic structural diagram of a composite structure assembly provided by the present disclosure.

As illustrated in Fig. 14, the composite structure assembly 40 includes a plurality of flexible neural electrode composite structures 410 (for example, three flexible neural electrode composite structures as illustrated in the figure), and each flexible neural electrode composite structure 410 has the same configuration as the flexible neural electrode composite structure 100 illustrated in Fig. 1.

For example, the composite structure assembly 40 further includes a connection part 420 having a plurality of conductive wires. Each conductive wire is connected between the electrode site of the flexible neural electrode and the pad (not illustrated) to realize signal transmission.

In the composite structure assembly provided by the embodiment of the present disclosure, since the auxiliary implantation end and the auxiliary structure are fixed by the fixture for each flexible neural electrode composite structure, the auxiliary implantation assembly and the plurality of flexible neural electrodes are fixed together. In this way, a plurality of flexible neural electrodes can be implanted into the target tissue at the same time during the implantation process of the flexible neural electrodes.

Compared with the implantation method in which the electrodes are implanted individually one by one, on the one hand, it shortens the implantation time and reduces the implantation difficulty, thus realizing the implantation of a flexible neural electrode array in a high-throughput and high-coverage manner; on the other hand, before the flexible neural electrodes are implanted, the flexible neural electrodes and the auxiliary implantation assembly having been assembled are fixed or connected together by a fixture, so that the operation of on-site assembling the flexible neural electrodes with the auxiliary implantation assembly during implantation is omitted, the implantation efficiency is improved, and the operation time is shortened; in addition, since the flexible neural electrodes and the auxiliary implantation assembly have been assembled into an integrated structure, it is convenient for transportation and usage.

At least one embodiment of the present disclosure also provides an implantation method of flexible neural electrodes adopting the flexible neural electrode composite structure described in any of the previous embodiments.

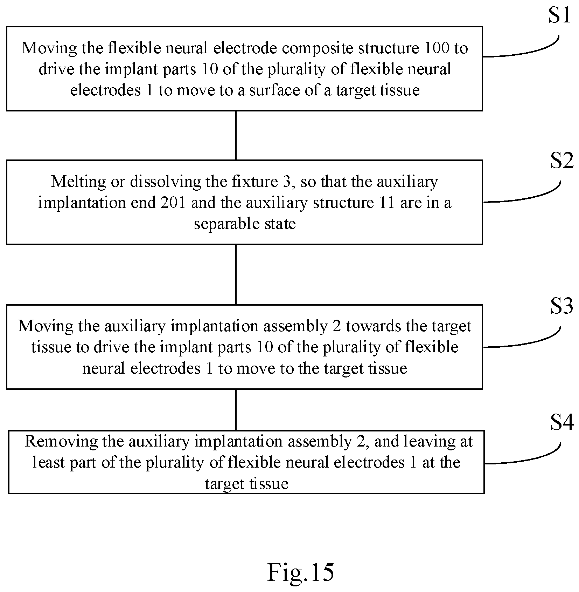

Fig. 15 is an implantation method of flexible neural electrodes adopting the flexible neural electrode composite structure of Fig. 1 as provided by the present disclosure.

With reference to Fig. 1 and Fig. 15, the above implantation method includes:
S1, moving the flexible neural electrode composite structure 100 to drive the implant parts 10 of the plurality of flexible neural electrodes 1 to move to a surface of a target tissue;
S2, melting or dissolving the fixture 3, so that the auxiliary implantation end 201 and the auxiliary structure 11 are in a separable state;
S3, moving the auxiliary implantation assembly 2 towards the target tissue to drive the implant parts 10 of the plurality of flexible neural electrodes 1 to move to the target tissue; and
S4: removing the auxiliary implantation assembly 2, and leaving at least part of the plurality of flexible neural electrodes 1 at the target tissue.

For example, when moving the auxiliary implantation assembly 2 toward the target tissue, the plurality of implant parts 10 of the plurality of flexible neural electrodes 1 are moved to the target tissue at the same time. When removing the auxiliary implantation assembly 2, the auxiliary implantation assembly 2 can be lifted so as to be separated from the flexible neural electrode(s) 1 left on the target tissue, so that the auxiliary implantation assembly 2 can be reused or directly discarded. The flexible neural electrode(s) left at the target tissue may be part or all of the plurality of flexible neural electrodes 1. When all the flexible neural electrodes 1 are left at the target tissue, more electrode sites may be provided on the target tissue.

As illustrated in Fig. 1, when each implant part 10 is a stretchable spiral structure, the auxiliary structure 11 is located on the stretchable spiral structure. In this way, when moving the auxiliary implantation assembly 2 towards the target tissue to drive the plurality of implant parts 10 of the plurality of flexible neural electrodes 1 to move to the target tissue, the auxiliary implantation end 201 can be utilized to push the auxiliary structure 11 downwardly, so that the stretchable spiral structure can be unfolded in the z direction.

As illustrated in Fig. 7, each implant part 10a has a linear structure, and the auxiliary structure 11a is located on the linear structure. In this way, when moving the auxiliary implantation assembly 2a towards the target tissue to drive the plurality of implant parts 10a of the plurality of flexible neural electrodes 1a to move to the target tissue, the auxiliary implantation end 201a can be utilized to push the auxiliary structure 11a downwardly to drive the linear structure to move to the target tissue. Compared with the spiral structure illustrated in Fig. 1, in the embodiment of Fig. 7, it is necessary to push the rear end of the flexible neural electrode 1a along with the implantation of the front end, or it is necessary to fold the rear end of the flexible neural electrode 1a to reserve a certain length before implantation, thereby reserving an implantation space for the whole implantation of the flexible neural electrode.

In the implantation method of flexible neural electrodes provided by the embodiment of the present disclosure, a plurality of flexible neural electrodes can be implanted into the target tissue at the same time. Compared with the implantation method in which the electrodes are implanted individually one by one, on the one hand, it shortens the implantation time and reduces the implantation difficulty, thus realizing the implantation of a flexible neural electrode array in a high-throughput and high-coverage manner; on the other hand, before the flexible neural electrodes are implanted, the flexible neural electrodes and the auxiliary implantation assembly having been assembled are fixed or connected together by a fixture, so that the operation of on-site assembling the flexible neural electrodes with the auxiliary implantation assembly during implantation is omitted, the implantation efficiency is improved, and the operation time is shortened; in addition, since the flexible neural electrodes and the auxiliary implantation assembly have been assembled into an integrated structure, it is convenient for transportation and usage.

In one example, the implantation method of flexible neural electrodes includes the following steps:
(1) Anesthesia of rat: a healthy SPF-level rat is anesthetized by injecting Pentobarbital sodium in accordance with the standard of 0.01 g/mL; after deeply anesthetized, the rat is fixed on the stereo locator, and then gas anesthesia is carried out with anesthetic for small animals; a cotton swab is dipped into iodophor and then applied onto the head of the rat for disinfection, and the hairs of the rat are cut off; the scalp of the rat is cut off along a middle seam with scissors, and the surface tissue of the skull is cleaned to expose the cleaned skull.
(2) Opening of cranial window: a rectangular cranial window of 5 mm×7 mm is formed by using a cranial drill at positions which are 2.5 mm in the front and back of the rat's anterior fontanel and 3.5 mm at the left and right of the midline, and the endocranium is carefully removed to provide a place for implanting flexible neural electrodes.
(3) Electrode implantation: the flexible neural electrode composite structure prepared in any of the previous embodiments and pre-fixed by a PVA film is fixed on the stereo locator, and is moved to gradually approach the cranial window in the surface of the rat brain; after the PVA film of the flexible neural electrode array is in contact with the surface of the brain, physiological saline is dropped into the gap between the PVA film and the silicon wafer to dissolve the PVA film; after the PVA film is dissolved and the flexible neural electrodes are completely released, the electrode implantation begins, and the descending speed of the clamping rod of the stereo locator is about 20 µm/s. When the descended height is about 1 mm, i.e., the height measured from the time the tip of the optical fiber contacts the surface of the brain, the clamping rod is further descended until the implantation depth reaches 1.5 mm, and then the implantation is stopped; the auxiliary implantation assembly is lifted in a speed of about 100 µm/s to complete the electrode implantation; finally, the hole in the skull is sealed with an isolation glue, and dental cement is applied around the electrodes and is applied between the electrodes and the iron-made carrier for fixation.

At least one embodiment of the present disclosure also provides an implantation method of flexible neural electrodes adopting the composite structure assembly described in the previous embodiment, which includes: implanting a plurality of groups of flexible neural electrodes into a target tissue by using a plurality of flexible neural electrode composite structures, and each group of flexible neural electrodes includes a plurality of flexible neural electrodes.

Referring to Fig. 14, the implantation method includes: sequentially implanting a first group of flexible neural electrodes 431, a second group of flexible neural electrodes 432 and a third group of flexible neural electrodes 433 into a target tissue by using a plurality of flexible neural electrode composite structures 410. Each group of flexible neural electrodes includes, for example, twelve flexible neural electrodes. Alternatively, the first group of flexible neural electrodes 431, the second group of flexible neural electrodes 432 and the third group of flexible neural electrodes 433 may also be implanted into the target tissue at the same time by using a plurality of flexible neural electrode composite structures 410. Through the above method, the implantation efficiency can be improved and the implantation time can be shortened.

At least one embodiment of the present disclosure also provides an auxiliary implantation assembly. For example, as illustrated in Fig. 5, the auxiliary implantation assembly 2 includes an auxiliary fixing member 21 including at least one auxiliary fixing plate 211; and a plurality of auxiliary implantation needles 20 configured to be connected with the at least one auxiliary fixing plate 211. The extension direction (the z direction as illustrated in the figure) of the plurality of auxiliary implantation needles 20 is not parallel to the plane (the xy plane as illustrated in the figure) where the at least one auxiliary fixing plate 211 is located.

For example, at least one auxiliary fixing plate 211 is detachably or fixedly connected with a plurality of auxiliary implantation needles 20. For example, the at least one auxiliary fixing plate 211 is provided with an opening 212, and the plurality of auxiliary implantation needles 20 each include a fixing end 202 arranged in opposite to the auxiliary implantation end 201, and the fixing end 202 is configured to pass through the opening 212 to be connected with the at least one auxiliary fixing plate 211.

For example, as illustrated in Fig. 6, the auxiliary implantation assembly 2 further includes an adhesive 22, and at least a part of the adhesive 22 is located between the at least one auxiliary fixing plate and the plurality of auxiliary implantation needles 20.

By using the auxiliary implantation assembly provided by the embodiment of the present disclosure, the implantable flexible neural electrode array can be transferred and implanted as a whole. Compared with the implantation method in which the electrodes are implanted individually one by one, the implantation and transfer method of electrode array can greatly improve the efficiency of transfer, release and implantation, reduce the time consumption of implantation, and provide a simple and efficient method for large-scale implantation of implantable flexible neural electrode array. In the implantation method provided by the embodiment of the present disclosure, the implantation speed is 1~200 µm/s (micron/s), such as 1 µm/s, 5 µm/s, 10 µm/s, 15 µm/s, 20 µm/s, 30 µm/s, 50 µm/s, 100 µm/s and 200 µm, for example, 10µm/s~20µm/s.

In the present disclosure, the following points need to be explained:
(1) The drawings of the embodiments of the present disclosure only refer to the structures related to the embodiment of the present disclosures, and other structures can refer to the general designs.
(2) Without conflict, the embodiments of the present disclosure and the features in the embodiments can be combined with each other to obtain new embodiment(s).

What have been described above merely are exemplary embodiments of the present disclosure, and are not used to limit the scope of protection of the present disclosure, which is determined by the appended claims.

## Claims

1. A flexible neural electrode composite structure (100), comprising:
a plurality of flexible neural electrodes (1), each of the plurality of flexible neural electrodes (1) comprising an implant part (10) and an auxiliary structure (11) provided on the implant part (10);
an auxiliary implantation assembly (2) comprising a plurality of auxiliary implantation needles (20) in one-to-one correspondence with the plurality of flexible neural electrodes (1), each of the plurality of auxiliary implantation needles (20) comprising an auxiliary implantation end (201) located close to one end of a corresponding flexible neural electrode (1), and the auxiliary implantation end (201) being configured to be assembled with the auxiliary structure (11); and
a fixture (3) configured to fix the auxiliary implantation end (201) and the auxiliary structure (11) which have been assembled,
**characterized in that**, the implant part (10) of each of the plurality of flexible neural electrodes (1) is a stretchable spiral structure.

2. The flexible neural electrode composite structure according to claim 1, wherein an extension direction of the plurality of auxiliary implantation needles (20) is not parallel to an extension direction of a plurality of implant parts (10) of the plurality of flexible neural electrodes (1).

3. The flexible neural electrode composite structure according to claim 2, wherein the extension direction of the plurality of auxiliary implantation needles (20) is not parallel to a plane where a plurality of spiral structures is located,
wherein the auxiliary structure (11) is located at an end of the stretchable spiral structure.

4. The flexible neural electrode composite structure according to claim 1, wherein the auxiliary implantation end (201) and the auxiliary structure (11) are configured to be assembled with each other by way of plugging-in,
wherein the auxiliary implantation end (201) is configured to be partially or completely inserted into the auxiliary structure (11).

5. The flexible neural electrode composite structure according to claim 1, wherein the auxiliary implantation assembly (2) further comprises:
an auxiliary fixing member (21) located at one side of the plurality of auxiliary implantation needles (20) away from the plurality of flexible neural electrodes (1) and configured to fix the plurality of auxiliary implantation needles (20).

6. The flexible neural electrode composite structure according to claim 5, wherein the auxiliary fixing member (21) comprises an auxiliary fixing plate (211), and a plane where the auxiliary fixing plate (211) is located is perpendicular to an extension direction of the plurality of auxiliary implantation needles (20).

7. The flexible neural electrode composite structure according to claim 6, wherein the auxiliary fixing plate (211) and the plurality of auxiliary implantation needles (20) are configured to be detachably or fixedly connected,
wherein the auxiliary fixing plate (211) is provided with a plurality of openings (212), and the plurality of auxiliary implantation needles (20) comprise fixing ends (202) away from the auxiliary implantation ends (201) along the extension direction of the plurality of auxiliary implantation needles (20), and the fixing ends (202) are configured to pass through the plurality of openings (212) to be connected with the auxiliary fixing plate (211).

8. The flexible neural electrode composite structure according to claim 1, wherein at least part of the fixture (3) is located between the auxiliary implantation end (201) and the auxiliary structure (11) to maintain a relative position between the auxiliary implantation end (201) and the auxiliary structure (11),
wherein the fixture (3) comprises one or more selected from a group consisting of a photo-meltable material, a thermal-meltable material, a liquid-swellable material and a liquid-dissolvable material.

9. A manufacturing method of a flexible neural electrode composite structure (100), comprising:
providing a plurality of flexible neural electrodes (1), wherein each of the plurality of flexible neural electrodes (1) comprises an implant part (10) and an auxiliary structure (11) formed on the implant part (10);
forming an auxiliary implantation assembly (2), wherein the auxiliary implantation assembly (2) comprises a plurality of auxiliary implantation needles (20), and each of the plurality of auxiliary implantation needles (20) comprises an auxiliary implantation end (201) located at one side of the auxiliary implantation needle (20) close to the plurality of flexible neural electrodes (1);
assembling the auxiliary implantation end (201) with the auxiliary structure (11); and
fixing the auxiliary implantation end (201) and the auxiliary structure (11) which have been assembled,
**characterized in that**, the implant part (10) of each of the plurality of flexible neural electrodes (1) is a stretchable spiral structure.

10. The manufacturing method according to claim 9, wherein before assembling the auxiliary implantation end with the auxiliary structure, the manufacturing method further comprises:
aligning the auxiliary implantation end with the auxiliary structure.

11. The manufacturing method according to claim 10, wherein aligning the auxiliary implantation end with the auxiliary structure comprises:
aligning at least one auxiliary implantation end among a plurality of auxiliary implantation ends with at least one auxiliary structure among a plurality of auxiliary structures.

12. The manufacturing method according to claim 9, wherein the fixing the auxiliary implantation end and the auxiliary structure which have been assembled comprises:
forming a fixture at least at a joint between the auxiliary implantation end and the auxiliary structure.

## Patentansprüche

1. Flexible neuronale Elektroden-Verbundstruktur (100), umfassend:
eine Mehrzahl flexibler neuraler Elektroden (1), wobei jede der Mehrzahl flexibler neuraler Elektroden (1) einen Implantatteil (10) und eine an dem Implantatteil (10) vorgesehene Hilfsstruktur (11) umfasst;
eine Hilfsimplantationsanordnung (2), die eine Mehrzahl von Hilfsimplantationsnadeln (20) in Eins-zu-eins-Zuordnung zu der Mehrzahl flexibler neuraler Elektroden (1) umfasst, wobei jede der Mehrzahl von Hilfsimplantationsnadeln (20) ein Hilfsimplantationsende (201) umfasst, das sich nahe einem Ende einer zugehörigen flexiblen neuralen Elektrode (1) befindet, und wobei das Hilfsimplantationsende (201) dazu ausgelegt ist, mit der Hilfsstruktur (11) zusammengebaut zu werden; und
eine Fixiereinrichtung (3), die dazu ausgelegt ist, das zusammengebaute Hilfsimplantationsende (201) und die Hilfsstruktur (11) zu fixieren,
**dadurch gekennzeichnet, dass** der Implantatteil (10) jeder der Mehrzahl flexibler neuraler Elektroden (1) eine dehnbare Spiralstruktur ist.

2. Flexible neuronale Elektroden-Verbundstruktur nach Anspruch 1, wobei eine Erstreckungsrichtung der Mehrzahl von Hilfsimplantationsnadeln (20) nicht parallel zu einer Erstreckungsrichtung einer Mehrzahl von Implantatteilen (10) der Mehrzahl flexibler neuraler Elektroden (1) verläuft.

3. Flexible neuronale Elektroden-Verbundstruktur nach Anspruch 2, wobei die Erstreckungsrichtung der Mehrzahl von Hilfsimplantationsnadeln (20) nicht parallel zu einer Ebene verläuft, in der sich eine Mehrzahl von Spiralstrukturen befindet,
wobei die Hilfsstruktur (11) an einem Ende der dehnbaren Spiralstruktur angeordnet ist.

4. Flexible neuronale Elektroden-Verbundstruktur nach Anspruch 1, wobei das Hilfsimplantationsende (201) und die Hilfsstruktur (11) dazu ausgelegt sind, durch die Steckverbindung miteinander zusammengebaut zu werden,
wobei das Hilfsimplantationsende (201) dazu ausgelegt ist, teilweise oder vollständig in die Hilfsstruktur (11) eingeführt zu werden.

5. Flexible neuronale Elektroden-Verbundstruktur nach Anspruch 1, wobei die Hilfsimplantationsanordnung (2) ferner Folgendes umfasst:
ein Hilfsbefestigungselement (21), das sich auf einer der Mehrzahl von Hilfsimplantationsnadeln (20) abgewandten Seite der Mehrzahl flexibler neuraler Elektroden (1) befindet und dazu ausgelegt ist, die Mehrzahl von Hilfsimplantationsnadeln (20) zu fixieren.

6. Flexible neuronale Elektroden-Verbundstruktur nach Anspruch 5, wobei das Hilfsbefestigungselement (21) eine Hilfsbefestigungsplatte (211) umfasst, und wobei eine Ebene, in der sich die Hilfsbefestigungsplatte (211) befindet, senkrecht zu einer Erstreckungsrichtung der Mehrzahl von Hilfsimplantationsnadeln (20) verläuft.

7. Flexible neuronale Elektroden-Verbundstruktur nach Anspruch 6, wobei die Hilfsbefestigungsplatte (211) und die Mehrzahl von Hilfsimplantationsnadeln (20) lösbar oder fest miteinander verbunden sind,
wobei die Hilfsbefestigungsplatte (211) mit einer Mehrzahl von Öffnungen (212) versehen ist, und wobei die Mehrzahl von Hilfsimplantationsnadeln (20) Befestigungsenden (202) umfasst, die entlang der Erstreckungsrichtung der Mehrzahl von Hilfsimplantationsnadeln (20) von den Hilfsimplantationsenden (201) abgewandt sind, und wobei die Befestigungsenden (202) dazu ausgelegt sind, durch die Mehrzahl von Öffnungen (212) hindurchgeführt zu werden, um mit der Hilfsbefestigungsplatte (211) verbunden zu werden.

8. Flexible neuronale Elektroden-Verbundstruktur nach Anspruch 1, wobei zumindest ein Teil der Fixiereinrichtung (3) zwischen dem Hilfsimplantationsende (201) und der Hilfsstruktur (11) angeordnet ist, um eine Relativposition zwischen dem Hilfsimplantationsende (201) und der Hilfsstruktur (11) aufrechtzuerhalten,
wobei die Fixiereinrichtung (3) ein oder mehrere Elemente umfasst, die aus einer Gruppe ausgewählt sind, die aus einem photo-schmelzbaren Material, einem thermoschmelzbaren Material, einem flüssigkeitsquellbaren Material und einem flüssigkeitslöslichen Material besteht.

9. Herstellungsverfahren für eine flexible neuronale Elektroden-Verbundstruktur (100), umfassend:
Bereitstellen einer Mehrzahl flexibler neuraler Elektroden (1), wobei jede der Mehrzahl flexibler neuraler Elektroden (1) einen Implantatteil (10) und eine auf dem Implantatteil (10) ausgebildete Hilfsstruktur (11) umfasst;
Ausbilden einer Hilfsimplantationsanordnung (2), wobei die Hilfsimplantationsanordnung (2) eine Mehrzahl von Hilfsimplantationsnadeln (20) umfasst, und wobei jede der Mehrzahl von Hilfsimplantationsnadeln (20) ein Hilfsimplantationsende (201) umfasst, das sich auf einer den Mehrzahl flexibler neuraler Elektroden (1) zugewandten Seite der Hilfsimplantationsnadel (20) befindet;
Zusammenbauen des Hilfsimplantationsendes (201) mit der Hilfsstruktur (11); und
Fixieren des zusammengebauten Hilfsimplantationsendes (201) und der Hilfsstruktur (11),
**dadurch gekennzeichnet, dass** der Implantatteil (10) jeder der Mehrzahl flexibler neuraler Elektroden (1) eine dehnbare Spiralstruktur ist.

10. Herstellungsverfahren nach Anspruch 9, wobei vor dem Zusammenbauen des Hilfsimplantationsendes mit der Hilfsstruktur das Herstellungsverfahren ferner Folgendes umfasst:
Ausrichten des Hilfsimplantationsendes mit der Hilfsstruktur.

11. Herstellungsverfahren nach Anspruch 10, wobei das Ausrichten des Hilfsimplantationsendes mit der Hilfsstruktur Folgendes umfasst:
Ausrichten zumindest eines Hilfsimplantationsendes unter einer Mehrzahl von Hilfsimplantationsenden mit zumindest einer Hilfsstruktur unter einer Mehrzahl von Hilfsstrukturen.

12. Herstellungsverfahren nach Anspruch 9, wobei das Fixieren des zusammengebauten Hilfsimplantationsendes und der Hilfsstruktur Folgendes umfasst:
Ausbilden einer Fixiereinrichtung zumindest an einer Verbindungsstelle zwischen dem Hilfsimplantationsende und der Hilfsstruktur.

## Revendications

1. Structure composite d'électrode neuronale flexible (100), comprenant :
une pluralité d'électrodes neuronales flexibles (1), chacune de la pluralité d'électrodes neuronales flexibles (1) comprenant une portion d'implantation (10) et une structure auxiliaire (11) prévue sur la portion d'implantation (10) ;
un ensemble d'implantation auxiliaire (2) comprenant une pluralité d'aiguilles d'implantation auxiliaires (20) en correspondance biunivoque avec la pluralité d'électrodes neuronales flexibles (1), chacune de la pluralité d'aiguilles d'implantation auxiliaires (20) comprenant une extrémité d'implantation auxiliaire (201) située à proximité d'une extrémité d'une électrode neuronale flexible (1) correspondante, et l'extrémité d'implantation auxiliaire (201) étant configurée pour être assemblée avec la structure auxiliaire (11) ; et
un élément de fixation (3) configuré pour fixer l'extrémité d'implantation auxiliaire (201) et la structure auxiliaire (11) qui ont été assemblées,
**caractérisée en ce que** la portion d'implantation (10) de chacune de la pluralité d'électrodes neuronales flexibles (1) est une structure hélicoïdale extensible.

2. Structure composite d'électrode neuronale flexible selon la revendication 1, dans laquelle une direction d'extension de la pluralité d'aiguilles d'implantation auxiliaires (20) n'est pas parallèle à une direction d'extension d'une pluralité de portions d'implantation (10) de la pluralité d'électrodes neuronales flexibles (1).

3. Structure composite d'électrode neuronale flexible selon la revendication 2, dans laquelle la direction d'extension de la pluralité d'aiguilles d'implantation auxiliaires (20) n'est pas parallèle à un plan où se trouve une pluralité de structures en spirale,
dans laquelle la structure auxiliaire (11) est située à une extrémité de la structure en spirale extensible.

4. Structure composite d'électrode neuronale flexible selon la revendication 1, dans laquelle l'extrémité d'implantation auxiliaire (201) et la structure auxiliaire (11) sont configurées pour être assemblées l'une avec l'autre par emboîtement,
dans laquelle l'extrémité d'implantation auxiliaire (201) est configurée pour être insérée partiellement ou complètement dans la structure auxiliaire (11).

5. Structure composite d'électrode neuronale flexible selon la revendication 1, dans laquelle l'ensemble d'implantation auxiliaire (2) comprend en outre :
un élément de fixation auxiliaire (21) situé à un côté de la pluralité d'aiguilles d'implantation auxiliaires (20) éloigné de la pluralité d'électrodes neuronales flexibles (1), et configuré pour fixer la pluralité d'aiguilles d'implantation auxiliaires (20).

6. Structure composite d'électrode neuronale flexible selon la revendication 5, dans laquelle l'élément de fixation auxiliaire (21) comprend une plaque de fixation auxiliaire (211), et un plan où se trouve la plaque de fixation auxiliaire (211) est perpendiculaire à une direction d'extension de la pluralité d'aiguilles d'implantation auxiliaires (20).

7. Structure composite d'électrode neuronale flexible selon la revendication 6, dans laquelle la plaque de fixation auxiliaire (211) et la pluralité d'aiguilles d'implantation auxiliaires (20) sont configurées pour être reliées de manière amovible ou fixe,
dans laquelle la plaque de fixation auxiliaire (211) est pourvue d'une pluralité d'ouvertures (212), et la pluralité d'aiguilles d'implantation auxiliaires (20) comprend des extrémités de fixation (202) éloignées des extrémités d'implantation auxiliaires (201) selon la direction d'extension de la pluralité d'aiguilles d'implantation auxiliaires (20), et les extrémités de fixation (202) sont configurées pour passer à travers la pluralité d'ouvertures (212) afin d'être reliées à la plaque de fixation auxiliaire (211).

8. Structure composite d'électrode neuronale flexible selon la revendication 1, dans laquelle au moins une partie de l'élément de fixation (3) est située entre l'extrémité d'implantation auxiliaire (201) et la structure auxiliaire (11) pour maintenir une position relative entre l'extrémité d'implantation auxiliaire (201) et la structure auxiliaire (11),
dans laquelle l'élément de fixation (3) comprend un ou plusieurs matériaux choisis dans un groupe constituant à un matériau photofondable, un matériau thermofondable, un matériau gonflable par liquide et un matériau soluble dans un liquide.

9. Procédé de fabrication d'une structure composite d'électrode neuronale flexible (100), comprenant :
fournir une pluralité d'électrodes neuronales flexibles (1), chacune de la pluralité d'électrodes neuronales flexibles (1) comprenant une portion d'implantation (10) et une structure auxiliaire (11) formée sur la portion d'implantation (10) ;
former un ensemble d'implantation auxiliaire (2), l'ensemble d'implantation auxiliaire (2) comprenant une pluralité d'aiguilles d'implantation auxiliaires (20), et chacune de la pluralité d'aiguilles d'implantation auxiliaires (20) comprenant une extrémité d'implantation auxiliaire (201) située à un côté de l'aiguille d'implantation auxiliaire (20) à proximité de la pluralité d'électrodes neuronales flexibles (1) ;
assembler l'extrémité d'implantation auxiliaire (201) avec la structure auxiliaire (11) ; et
fixer l'extrémité d'implantation auxiliaire (201) et la structure auxiliaire (11) qui ont été assemblées,
**caractérisé en ce que** la portion d'implantation (10) de chacune de la pluralité d'électrodes neuronales flexibles (1) est une structure hélicoïdale extensible.

10. Procédé de fabrication selon la revendication 9, dans lequel, avant d'assembler l'extrémité d'implantation auxiliaire avec la structure auxiliaire, le procédé de fabrication comprend en outre :
aligner l'extrémité d'implantation auxiliaire avec la structure auxiliaire.

11. Procédé de fabrication selon la revendication 10, dans lequel l'alignement de l'extrémité d'implantation auxiliaire avec la structure auxiliaire comprend :
aligner au moins une extrémité d'implantation auxiliaire parmi une pluralité d'extrémités d'implantation auxiliaires avec au moins une structure auxiliaire parmi une pluralité de structures auxiliaires.

12. Procédé de fabrication selon la revendication 9, dans lequel la fixation de l'extrémité d'implantation auxiliaire et de la structure auxiliaire qui ont été assemblées comprend :
former un élément de fixation au moins à une jonction entre l'extrémité d'implantation auxiliaire et la structure auxiliaire.
